Europäisches Patentamt

European Patent Office

Office européen des brevets

(19)

(11) Publication number: **0 443 511 A2**

# EUROPEAN PATENT APPLICATION

(21) Application number: 91102329.9

(22) Date of filing: 19.02.91

(51) Int. Cl.⁵: **C12P 21/08**, G01N 33/577, G01N 33/68, C12N 5/12

(30) Priority: 21.02.90 JP 40689/90
29.01.91 JP 9393/91

(43) Date of publication of application:
28.08.91 Bulletin 91/35

(84) Designated Contracting States:
DE FR GB IT

(71) Applicant: MERCIAN CORPORATION
5-8, Kyobashi 1-chome
Chuo-ku, Tokyo(JP)

(72) Inventor: Matsumura, Sueo
1001-10, Hachinohe, Nabeshima-cho
Saga-shi, Saga-ken(JP)
Inventor: Kimura, Akihiko
4-10-10-202, Funatsu-cho
Wakayama-shi, Wakayama-ken(JP)

(74) Representative: Werner, Hans-Karsten, Dr. et
al
Deichmannhaus am Hauptbahnhof
W-5000 Köln 1(DE)

(54) A monoclonal antibody specific for myosin in the brain.

(57) A monoclonal antibody specifically reacting with myosin isozymes existing in human brain. A monoclonal antibody specifically reacting with myosin isozymes of bovine brain tissue. A monoclonal anibody capable of reacting on several kinds of myosins including myosins present in bovine brain. Also disclosed are a hybridoma producing a monoclonal antibody specifically recognizing myosin isozymes existing in human brain; a hybridoma producing a monoclonal antibody specifically recognizing myosin isozymes existing in bovine brain; and a hybridoma producing a monoclonal anibody capable of reacting on several kinds of myosins including myosins present in bovine brain.

EP 0 443 511 A2

# A MONOCLONAL ANTIBODY SPECIFIC FOR MYOSIN IN THE BRAIN.

## BACKGROUND OF THE INVENTION

### Field of the Invention

The present invention relates to a monoclonal antibody specific for a myosin isozyme in the brain, a monoclonal antibody capable of reacting with myosin isozymes in the brain, and hybridomas producing said monoclonal antibodies.

## DESCRIPTION OF THE RELATED ART

Myosins have been found in muscule such as, for example, skeletal muscle, cardiac muscle, and smooth muscle, and they have been known for a long time to participate in muscular contraction. Recently, myosins have been found in almost all kinds of cells making up non-muscular systems as well as in myocytes. These myosins are considered to be involved in various kinds of cell movements such as, for example, cytoplasmic division, i.e., cell division, which is connected with cell membrane movement, secretion, phagocytosis, and capping of a lot of receptors.

Among these myosins, myosins of skeletal muscle, cardiac muscle, or smooth muscle are easily distinguishable from each other by the differences in structure and properties. Antibodies capable of distinguishing these isozymic myosins of different muscles have been obtained. Generally, however, it has been found to be difficult to distinguish myosins existing in non-muscular systems from myosins present in smooth muscle since the properties and structures of the former myosins are similar to those of the latter myosins. Furthermore, it has been found to be more difficult to distinguish myosin isozymes present in various non-muscular systems since they are structurally quite similar to one another.

In neurocytes, myosins are believed to participate in the formation and elongation of the axis-cylinder as well as in the above-mentioned cell movements. Since all of these cell movements are related to the fundamental functions of the cells, it is likely that the qualitative or quantitative alteration of myosins existing in neurocytes cause diseases such as, for example, various kinds of brain tumors, senile encephalatrophy, and Alzheimer's encephalatrophy accompanied with the qualitative alteration of neurocytes.

Therefore, if a monoclonal antibody specifically recognizing myosins existing in cerebral neurocytes or a monoclonal antibody capable of reacting on several myosins including myosins existing in cerebral neurocytes is obtained, the monoclonal antibody would be useful for diagnosing cerebral nervous diseases such as those mentioned above. The monoclonal antibodies would also be useful to diagnose brain death or brain contusion.

## SUMMARY OF THE INVENTION

An object of the present invention is to provide a monoclonal antibody specifically recognizing myosin isozymes of cerebral neurocytes.

Another object of the present invention is to provide a monoclonal antibody capable of reacting on several myosins including myosins of cerebral neurocytes.

More specifically, an object of the present invention is to provide a monoclonal antibody recognizing myosin isozymes of the human brain or bovine brain.

A further object of the present invention is to provide hybridomas producing said monoclonal antibodies.

The inventors of the present invention have conducted various studies to achieve the foregoing objects, and have found that the objects can be effectively attained by providing monoclonal antibodies, obtained from the cultivation of hybridomas prepared by using myosins of the human brain or bovine brain as antigens, which antibodies specifically recognize myosin or are capable of reacting with several myosins including myosins present in the human brain or bovine brain.

In accordance with the above object, the present invention provides a monoclonal antibody specifically reacting with a myosin isozyme of human brain tissue; a monoclonal antibody specifically reacting with myosin isozymes existing in bovine brain; and a monoclonal antibody capable of reacting with a several kinds of myosins including myosins of bovine brain tissue.

In accordance with other embodiments of the present invention, there is provided a hybridoma producing a monoclonal antibody specifically react on a myosin isozymes existing in the human brain; a hybridoma producing a monoclonal antibody specifically reacting with myosin isozymes of bovine brain tissue; and a hybridoma producing a monoclonal antibody reacting on several kinds of myosins including myosins of bovine brain tissue.

Further objects, features and advantages of the present invention will become apparent from the Description of the Preferred Embodiments which follows, when read in light of the attached Examples.

DETAILED DESCRIPTION OF THE PREFERRED EMBODIMENT

A detailed description will be provided hereinafter as to the method for preparing the hybridomas and monoclonal antibodies of the present invention.

Myosins of human cerebral neurocytes or bovine cerebral neurocytes used for preparing the monoclonal antibody of the present invention can be extracted and purified from human cerebrum or bovine cerebrum according to the method described by Matsumura (J. Biochem., 103: 237-246, 1988).

Myosins of bovine cerebral neurocytes preprared according to the above method have the molecular weight of about 500,000, which consist of two heavy chain components having the molecular weight of about 200,000 and four light chain components having the molecular weight of about 20,000. Myosins of human cerebral neurocytes have almost the same molecular weight and structure as those of bovine myosins.

The immunization of a mammal such as, for example, mouse, rat, rabbit, sheep, horse, or cow, may be carried out by administering the myosins, obtained according to the method described above, as antigens. An adjuvant such as, for example, Freund's complete adjuvant, may be used in the immunization.

When Freund's complete adjuvant is used, an antigenic suspension can be preprared by suspending the myosin and the adjuvant in a ratio of 1 : 1 to 1 : 10 (V/V), preferably 1 : 1 (V/V), in a 0.25-0.5 M aqueous sodium chloride solution so that the suspension contains the myosin in a concentration of 0.1 to 10 mg/ml, preferably 1 mg/ml.

The mammal can be immunized against brain myosin by administering the antigenic suspension to the mammal. When mouse is used for the immunization, a mouse can be effectively immunized by subcutaneous administration such as, for example, s.c. administration to the planta pedis, or intraperitoneal administration of the antigenic suspension from one to three times, preferably twice or three times to a mouse within an interval of from 1 to 2 weeks.

Antibody forming cells can be obtained by separating such antibody forming cells as, for example, lymphonodus lymphocytes, spleen cells, thymocytes, peripheral blood cells from the mammal immunized according to the method described above. When lymphonodus lymphocytes are used as antibody forming cells, the antibody forming cells can be prepared by extracting a lymphnode from the immunized mammal, washing the tissue with RPMI 1640 medium containing 15% fetal bovine serum (available from GIBCO, hereinafter referred to as NS-1 medium), mincing the lymphnode with scissors, separating lymphocytes by passing the lymphnode through a stainless mesh, followed by washing the lymphocytes with NS-1 medium by centrifuging the cells. Where spleen cells are used as antibody forming cells, the antibody forming cells can be prepared by a method comprising the steps of removing the spleen from the immunized mammal; washing the spleen with, for example, NS-1 medium; cutting the spleen into pieces in the medium; passing the tissue through a stainless mesh; and subsequently washing the spleen cells with the medium by centrifugation.

Myeloma cells used in the cell fusion process may be derived from various kinds of mammals such as, for example, mouse, rat, rabbit, and human. For example, myeloma cells which do not produce hypoxanthine-guanine-phosphoribosyl-transferase (HGPRT) may be used in the cell fusion process. Such myeloma cells, which do not produce HGPRT, are not able to synthesize nucleic acids and cannot grow in hypoxanthine-aminopterin-thymidine (HAT) medium unless they are fused with other cells, while a hybridoma obtained by the cell fusion of the myeloma cell with a lympocyte recovers the HGPRT activity and can grow in said medium by metabolizing hypoxanthines, although aminopterin inhibits the biosynthesis of nucleic acids. Thus, hybridomas can be selectively cultured in the above medium. Preferably, myeloma cells may be non-secreting myeloma cells. Examples of the myeloma cells include, for example, mouse myeloma $P_3/X63-Ag8U1(P_3U_1)$, $P_3/NS1-1-Ag4-1(NS-1)$, $P_3/X63-Ag8-6.5.3(X63.6.5.3)$, SP2/0-Ag14(SP2); rat myeloma 210.RCY3.Ag1.2.3(Y3.Ag1.2.3); and human myeloma SKO-007 and GM1500TG-A12.

Cell fusion may be carried out by mixing $10^7 - 10^8$ cells of the myeloma with antibody forming cells in a ratio of from 1 : 4 to 1 : 10 in a growth medium such as, for example, RPMI 1640, which is generally used for the cultivation of mammalian cells. A cell fusion accelerator such as, for example, polyethylene glycols (PEG) having an average molecular weight of 1,000 to 6,000, polyvinylalcohols, and Sendai virus may be used in the process. In general, the cell fusion process may be continued for 5 - 20 minutes, preferably for 6 minutes, at 25°C , preferably in the presence of PEG.

An elective cultivation in a selective medium may be carried out for separating hybridomas from the cell mixture obtained after the cell fusion process. For example, the elective cultivation may comprise the steps of diluting the cells with a medium such as, for example, NS-1 medium to obtain a cell-suspension containing $5 \times 10^6$ cells/ml; adding the cell suspension to the wells of a micro-titer plate so that each well contains about from $10^5$ to $10^6$ cells; adding a selective medium such as, for example, HAT medium to the wells; and incubating the cells while exchanging the selective medium in an appropriate interval of, for example, three days. In the case where $P_3$ /X63-Ag8U1($P_3U_1$ ) cells are used as myeloma cells, hybridomas can be saparated by incubating cell mixtures in HAT medium for about 10 to 14 days.

Selection of a hybridoma, which produces monoclonal antibodies specifically recognizing isozymic brain-myosins, may be carried out according to a known method such as, for example, an enzyme-linked immunosorbent assay (ELISA).

The ELISA process may comprise the steps of dissolving a sample containing brain myosins in a buffered solution such as, for example, phosphate-buffered saline (PBS) or sodium hydrogen carbonate buffer (pH 8.0); adding the solution onto a solid substrate such as, for example, soft plates made of polystyrene, polyvinyl, polycarbonate, polypropylene, polyacrylamide, silicone, dextran, or cellulose, glass plates, beads, sheets, wells, or tubes; standing the solid substrate, for example, overnight at 4 °C in order to complete the adsorption of the antigen; discarding the antigenic solution and washing the solid substrate with PBS; adding PBS which contains 1% bovine serum albumin (BSA); standing the solid matrix, for example, overnight at 4°C so that BSA blocks the remaining portion of the surface of the substrate not covered with the antigen; adding 50 $\mu$ l of the supernatant of the cultural medium containing hybridoma and standing the mixture, for example, at room temperature for 1 hour; washing the substrate three times with PBS, adding biotinylated anti-mouse immunoglobulin antiserum (second antigen) and standing the mixture for 1 hour at room temperature; washing the substrate three times with PBS followed by adding a solution of an enzyme linked with avidin; standing the mixture for 15 minutes at room temperature; washing the matrix four times with PBS; adding an enzymic substrate to the mixture for the color-forming of the sample solution; and spectrophotometrically or spectrofluorometrically measuring the sample to distinguish the culture medium containining hybridomas producing monoclonal antibodies having affinity with the antigen.

After distinguishing the culture medium containing antibody-forming cells according to the method described above, antibody-forming cells originating from a single hybridoma can be obtained by a known cloning procedure such as, for example, a limiting dilution analysis.

The monoclonal antibodies produced by said hybridoma can be obtained from the supernatant of the culture medium after the cultivation of the hybridoma using a culture flask or a culture bottle. Any medium suitable for the cultivation of mammalian cells such as, for example, RPMI 1640 medium containing 10 - 15% fetal bovine serum, or a serum-free medium, may be used for the cultivation. Methods or conditions for the cultivation of the hybridomas may be suitably choosen by those of ordinary skill in the art in view of prior art disclosures of methods for cultivating mammalian cells.

An example of a method for preparing a large amount of antibodies includes the method comprising the steps of administering mineral oil such as, for example, pristane (2,6,10,14-tetramethylpentadecane) intraperitoneally to a mammal such as, for example, the same sort of mammal from which the myeloma cells are derived; and followed by administering said hybridoma intraperitoneally to the mammal so that the cells can intensively multiply in the abdominal cavity. According to the method described above, said hybridomas form neoplastic ascites in 10 - 18 days from which monoclonal antibodies are released in serum and ascites at a high concentration of, for example, about 1 - 20 mg/ml.

Where a further purification of the monoclonal anibodies is required, the purification processes may comprise fractionating the ascites by using ammonium sulfate; and followed by subjecting the fraction obtained to chromatography such as, for example, DEAE-cellulose ion-exchange chromatography, affinity column chromatography using Sepharose-4B which is covalently bonded to protein G or brain-myosin, or gel-filtration column chromatography.

Examples of the hybridoma obtained according to the method described above include hybridomas BM-1 and BM-2 producing monoclonal antibodies specifically reacting with bovine brain myosin; and hybridoma HBM-1 producing a monoclonal antibody specifically reacting with human brain myosin. From these hybridomas, monoclonal antibodies BM-1, BM-2, and HEM-1 specifically reacting with isozymic myosins of brain tissue were obtained. The antibodies reacted with isozymic myosins present in cerebral neurocytes, but they did not react with myosins existing in smooth muscles, non-muscular systems except brain, cardiac muscles, or skeletal muscles. Examples of the hybridoma further include hybridomas BM-3 and BM-4 which produce monoclonal antibodies capable of reacting on myosins existing in bovine brain. The monoclonal antibody produced by hybridoma BM-3 was capable of reacting with myosins present in non-muscular system including cerebral neurocytes, but it did not react with myosins existing in muscular systems such as, for example, skeletal muscles, cardiac muscle, or smooth muscle. The monoclonal antibody produced by hybridoma BM-4 was capable of reacting with myosins present in non-muscular system including cerebral neurocytes and myosins present in smooth muscles, while it did not react with myosins existing in muscular systems such as skeletal muscle or cardiac muscle.

The above-described hybridomas BM-1, BM-2, BM-3, and BM-4 were deposited under the Budapest Treaty on February 1, 1990, with the Fermentation Research Institute, Higashi 1-1-3, Tsukuba, Ibaragi, Japan, under depository No. FERM BP-3229, FERM BP-3230, FERM BP-3231, and FERM BP-3232, respectively. Hybridoma HBM-1 was also deposited on December 26, 1990, with the same depository under No. FERM BP-3233. All restrictions on the availability to the public of the deposited microorganisms will be irrevocably removed upon the granting of a Patent hereon.

The monoclonal antibodies of the present invention produced by the hybridoma of the present invention are useful for diagnosing cerebral nervous diseases such as, for example, various kinds of brain tumors, senile encephalatrophy, and Alzheimer's encephalatrophy accompanied with the qualitative alteration of neurocytes. In addition, the monoclonal antibodies of the present invention are useful to diagnose brain death or brain contusion and also useful for the investigation of the generation or differentiation of cerebral nervous system.

The present invention will be further illustrated by the following Examples. The Examples are given by way of illustration only and are not construed as limiting.

EXAMPLES

Example 1: Preparation of hybridoma

Bovine cerebrum myosins were dissolved in 0.5 M aqueous sodium chloride solution at a concentration of $1\mu$ g/$\mu$ l and mixed with Freund's complete adjuvant in a ratio of 1 : 1 (V/V) to form a homogeneous emulsion. Of the emulsion, 100 $\mu$ l (equivalent to 50 $\mu$ g of myosin) was hypodermically injected into each planta of a 6 week-old BALB/c female mouse. After 9 days, both popliteal lymph nodes were enucleated and washed with NS-1 medium. Mouse myeloma $P_3U_1$ cells were washed with NS-1 medium and the lymphocytes obtained above were mixed with $P_3U_1$ cells in the ratio of 5 : 1. After centrifuging the mixture, the pellet obtained was washed with fetal bovine serum-free RPMI 1640 medium. 1 ml of 50% solution of PEG 1000 in RPMI 1640 medium was added gradually to the pellet in order to obtain 10 ml of a cell suspension, and the pellet was obtained by centrifuging the cell suspension. The pellet obtained was suspended in HAT medium containing 15% fetal bovine serum so that the resulting suspension contained lympocytes at a concentration of 5 X $10^5$ cells/0.1 ml. To each well of a 96-well plate, 0.1 ml of the cell suspension was added. After one day, 0.1 ml of HAT medium was added to the well. Thereafter, half of the cultural medium was exchanged with fresh HAT medium every three or four days. Hybridoma colony formation was observed in several wells for about 7 day's cultivation.

Of the supernatant of the cultural medium in a well in which the colony formation of hybridomas was observed, 50 $\mu$ l was added to the wells of a soft plate previously coated with bovine cerebrum myosin, or myosins prepared from bovine skeltal muscle, bovine aorta, bovine liver, bovine spleen, bovine thymus, bovine adrenal cortex, or bovine adrenal medulla. Hybridomas were selected by the ELISA method using avidin-D peroxidase (Vector) as peroxidase-conjugated avidin, hydrogen peroxide and o-phenylenediamine as an enzymic substrate and a color former. Hybridoma producing monoclonal antibodies were obtained from the supernatant of the cultural medium, which could react with myosins existing in bovine cerebrum or rat cerebrum, but could not react with myosins present in skeletal muscle, cardiac muscle, and aorta, and were less reactive with the myosins of non-muscular system. The hybridomas were subjected to further cloning by the limiting dilution method. In the above experiments, cerebrum myosins were purified according to the method described by Matsumura et al. (J. Biochem., 103: 237-246, 1988) so that the purity of the myosin was not less than 95%. Myosins were purified from liver, spleen, thymus, adrenal cortex, or adrenal medulla in similar manner described by Matsumura et al. so that the purity of each myosin was about 95%. Myosins existing in skeletal muscle, cardiac muscle, and aorta were purified according to the method described by Margossian et al.(Methods Enzymol. 85: 55-71, 1982), Siemankowsky et al.(J. Biol. Chem., 253: 8648-8658, 1978), and Persechini et al.(Biochem., 22: 470-476, 1983), respectively.

The following hybridomas were obtained in the experiment described above: hybridomas BM-1 and BM-2 producing the monoclonal antibodies BM-1 and BM-2, respectively, specifically recognizing brian myosins; hybridoma BM-3 producing the monoclonal antibody BM-3 capable of reacting with myosins of various kinds of non-muscular systems such as, for example, liver, spleen, thymus, adrenal cortex, or adrenal medulla, but not reacive with myosins present in skeletal muscle, cardiac muscle, and aorta; and hybridoma BM-4 producing the monoclonal antibody BM-4 capable of reacting with myosins of the aorta (smooth muscle) and other non-muscle systems, but not reactive with myosins present in skeletal muscle and cardiac muscle (striated muscle).

Example 2: The production of the monoclonal antibody

(a) The production of monoclonal antibody in the supernatant of the cultural medium was carried out as follows: hybridomas BM-1, BM-2, BM-3, and BM-4 were cultured in NS-1 medium by succesive scaling up cultivations using a 96-well plate, a 25-dl flask, and a 75-dl flask for each 2 days at 37 °C in a carbon-dioxide incubator and the supernatant of the cultural medium was collected.

(b) The production of monoclonal antibody in mouse ascites was carried out as follows: 0.5 ml of pristane (Aldrich) was injected intraperitoneally to 6-week old BALB/c mice and hybridomas BM-1, EM-2, BM-3, or BM-4 were intraperitoneally administered to the mice ($1 \times 10^7$ hybridomas/mouse) after 3 weeks. After abdominal hypertrophy was observed in one or two weeks, 5 ml of ascites which contained 10 mg/ml of monoclonal antibody was obtained from each mouse by abdominal section.

Example 3: Properties of the monoclonal antibody

(a) Tissue specificities of the anti-myosin monoclonal antibodies were as follows:

Monoclonal antibodies BM-1 and BM-2 had high reactivities with myosins extracted from brain systems such as, for example, cerebrum, cerebellum, and medulla oblongata as well as with myosins extracted from adrenal body, but they had little reactivity with myosins separated from other tissues. Monoclonal antibody BM-3 was not reactive with myosins extracted from muscular systems such as, for example, skeletal muscle, cardiac muscle, and smooth muscle, but it had high reactivity with myosins extracted from non-muscular systems including brain systems. Monoclonal antibody BM-4 had high reactivity with myosins extracted from smooth muscle and non-muscular systems, but it was not reactive with myosins extracted from skeletal muscle or cardiac muscle (striated muscle).

The results are summarized in Tables 1 and 2.

## Table 1

### Tissue Specificity of Monoclonal Antibodies

### Determined by ELISA Method.

| Tissue from which myosin was extracted | Monoclonal Antibody | | | |
|---|---|---|---|---|
| | BM-1 | BM-2 | BM-3 | BM-4 |
| cerebrum | 100 | 100 | 100 | 100 |
| cerebellum | 36 | 60 | 7 | 81 |
| liver | 0 | 8 | 132 | 110 |
| spleen | 0 | 0 | 72 | 110 |
| thymus | 0 | 7 | 149 | 116 |
| adrenal cortex | 13 | 47 | 172 | 93 |
| adrenal medulla | 5 | 32 | 132 | 108 |
| skeletal muscle | 0 | 0 | 0 | 0 |
| cardiac muscle | 0 | 0 | 0 | 0 |
| aorta (smooth muscle) | 0 | 0 | 5 | 143 |

1) The dilution ratio of the supernatants was 1/60 (BM-1), 1/8 (BM-2), 1/60 (BM-3), and 1/250 (BM-4), respectively.

2) Each well was coated with 0.5 $\mu$ g of purified myosin.

3) The reactivity of the peroxidase conjugated with cerebrum myosin ($A_{492}$ - $A_{620}$ / 30 min.) was indicated as 100% reactivity.

Table 2

Tissue Specificity of Monoclonal Antibodies

Determined by Dot Blot Method.

| Tissue from which myosin was obtained | Monoclonal Antibody | | | |
|---|---|---|---|---|
| | BM-1 | BM-2 | BM-3 | BM-4 |
| cerebrum | ++++ | ++++ | +++ | ++++ |
| cerebellum | +++ | +++ | + | ++ |
| medulla oblongata | + | + | + | ++ |
| liver | ± | ± | ++++ | ++++ |
| spleen | ± | ± | ++ | ++++ |
| thymus | — | — | ++++ | +++ |
| adrenal cortex | ++ | ++ | ++++ | ++++ |
| adrenal medulla | + | + | +++ | ++++ |
| skeletal muscle | — | — | — | — |
| cardiac muscle | — | — | — | — |
| aorta (smooth) | — | — | — | ++++ |

1) The dilution ratio of the supernatants was 1/100 (BM-1), 1/20 (BM-2), 1/200 (BM-3), and 1/200 (BM-4), respectively.

2) 5 $\mu$ l of purified myosin solution (0.5 $\mu$ g/$\mu$ l) was adsorbed directly to nitrocellulose membrane (equivalent ot 2.5 $\mu$ g myosin).

(b) Antigenic binding sites of myosin to the monoclonal antibodies

A myosin molecule consists of two spherical or ellipsoidal "head" portions and one lanky "tail" portion. Two heavy chain components constitute both head and tail portions of myosin, while four light chain components two by two constitute its head portions. The myosin molecule conjugated to monoclonal antibodies BM-1, BM-2, BM-3, and BM-4 in its tail portion and not in its head portion, i.e., a heavy component of myosin reacted with the monoclonal antibodies, while a light component did not react with the antibodies. Electronmicroscopic observation of the antibodies conjugated with myosin molecules, as well as an investigation of the reactivities of the antibodies with various kinds of fragments obtained by treating myosin with various proteases, revealed that the antigenic binding sites of myosin to antibodies BM-1, BM-2, and BM-3 lied on the head side of the tail of myosin, while antibody BM-4 conjugated on the center of the tail of myosin. Antibody BM-1 or BM-2 bind to a specific stereostructure of an antigen determinant which lies on the tail of myosin. The treatment of myosin by dodecyl sulfate or the like to alter the stereostructure of the myosin made the myosin impossible to conjugate with antibodies BM-1 or BM-2, while the treatment of myosin by formalin or ethanol did not decrease the reactivity of the antibodies with the myosin.

(c) Subclasses of the monoclonal antibodies were determined by the ELISA method and immuno-blotting method using a subclass kit available from Binding-Site, Co., Ltd. Subclasses of BM-1, BM-2, BM-3, and BM-4 were IgG2b, IgG1, IgG1, and IgM, respectively.

Example 4: Preparation of the hybridomas

Human cerebrum myosin was dissolved in 0.5 M aqueous sodium chloride solution at a concentration of 1 mg/ml and the solution was mixed with Freund's complete adjuvant in a ratio of 1 : 1 (V/V) to form a homogeneous emulsion. 100 $\mu$ l of the emulsion (equivalent to 50 $\mu$ g of myosin) was intraperitoneally injected in to a 6 week-old BALB/c female mouse, and after 21 days, an emulsion containing myosin and imcomplete adjuvant was injected intraperitoneally in the same manner described above. After additional 21 days, myosin suspended in saline was injected intraperitoneally into the mouse, and after 3 days, the spleen was enucleated and washed with NS-1 medium. The spleen was cut into pieces with a knife and spleen cells were obtained by passing the tissue through a stainless mesh and washed with NS-1 medium by centrifugation. Mouse myeloma $P_3U_1$ cells were washed with NS-1 medium and the spleen cells obtained above were mixed with $P_3U_1$ cells in the ratio of 5 : 1. After centrifuging the mixture, the pellet obtained was washed with fetal bovine serum-free RPMI 1640 medium. 1 ml of 50% solution of PEG 1000 in RPMI 1640 medium was added gradually to the pellet to obtain a cell suspension of the final volume of 10 ml. Pellet was obtained by centrifuging the cell suspension and the pellet obtained was suspended in HAT medium containing 15% fetal bovine serum so that the resulting suspension contained spleen cells at the concentration of $5 \times 10^5$ cells/0.1 ml. Of the cell suspension, 0.1 ml was added to each well of a 96-well micro-titer plate. After one day, 0.1 ml of HAT medium was added to the well. Thereafter, half of the cultural medium was exchanged with fresh HAT medium every three or four days. Hybridoma colony formation was observed in several wells for about 7 day's cultivation.

Of the supernatant of the cultural medium in the well in which the colony formation of hybridomas was observed, 50 $\mu$ l was added to the wells of a soft plate previously coated with human cerebrum myosin, or myosins extracted from human skeletal muscle, human cardiac muscle, human aorta, human liver, human spleen, or human thrombocyte. Hybridomas were selected by the ELISA method using avidin-peroxidase (Vector) as peroxidase-conjugated avidin, hydrogen peroxide and o-phenylenediamine as an enzymic substrate and a color forming agent. A hybridoma was thus obtained which produces the monoclonal antibodies, in the supernatant of the cultural medium, which could react with myosins of the human cerebrum, but did not react myosins present in human skeletal muscle, cardiac muscle, and aorta, and were less reactive with myosins of other non-muscular systems. The hybridomas selected were subjected to further cloning by the limiting dilution method. In the above experiments, cerebrum myosins were purified according to the method described by Matsumura et al. (J. Biochem., 103: 237-246, 1988) so that the purity of the myosin was not less than 95% . Myosins were purified from liver, spleen, or kidney in a similar manner described by Matsumura et al. The purity of each myosin was about 95%. Myosins was purified from human thrombocytes according to the methods described by Takashima (J. Biochem., 104: 1027 - 1035, 1982). Myosins exisiting in skeletal muscle, cardiac muscle, and aorta were purified according to the method described by Margossian et al.(Methods Enzymol. 85: 55-71, 1982), Siemankowsky et al.(J. Biol. Chem., 253: 8648-8658, 1978), and Persechini et al.(Biochem., 22: 470-476, 1983), respectively.

According to the procedure described above, hybridoma HBM-1, producing the monoclonal antibody specifically recognizing isozymic human brain-myosins, was obtained.

Example 5: Production of the monoclonal antibody

(a) The production of monoclonal antibody in the supernatant of the cultural medium was carried out as follows: hybridoma HBM-1 were cultured in NS-1 medium by succesive scaling up cultivations using a 96-well plate, a 25-dl flask, and a 75-dl flask for 2 days each at 37 $^\circ$C in a carbon-dioxide incubator wherein the supernatant of the cultural medium was collected.

(b) The production of monoclonal antibody in mouse ascites was carried out as follows: 0.5 ml of pristane (Aldrich) was injected intraperitoneally into 6-week old BALB/c mice and hybridoma HBM-1 was intraperitoneally administered to the mice ($1 \times 10^7$ hybridomas/mouse) after 3 weeks. After abdominal hypertrophy was observed in one or two weeks, 5 ml of ascites containing 10 mg/ml of monoclonal antibody was obtained from each mouse by abdominal section.

Experiment 6: Properties of the monoclonal antibody

(a) Tissue specificities of the anti-myosin monoclonal antibody were as follows: Monoclonal antibody HBM-1 had a high reactivity with myosins extracted from human cerebrum or human cerebellum, but had little reactivity with myosins extracted from other tissues. Monoclonal antibody HBM-1 was slightly reactive (5 - 10%) with myosins separated from bovine cerebrum, bovine cerebellum, and rat brain, but had no reactivity with myosins extracted from other bovine tissues such as, for example, skeletal muscle, cardiac muscle, aorta, thymus, liver, spleen, adrenal body, kidney, or testicle.

The results obtained in the experiment are summarized in Tables 3 and 4.

Table 3

Tissue Specificity of Monoclonal Antibody HBM-1

Determined by ELISA Method.

| Tissue from which myosin was extracted | Reactivity of HBM-1 (%) |
|---|---|
| cerebrum | 100 |
| cerebellum | 40 |
| liver | 0 |
| spleen | 0 |
| thrombocyte | 0 |
| aorta[4] | 6 |
| uterus[4] | 4 |
| skeletal muscle | 0 |
| cardiac muscle | 0 |

1) Each well was coated with 0.5 $\mu$ g of purified myosin.
2) The activity of the peroxidase conjugated with cerebrum myosin ($A_{492}$ - $A_{620}$ / 30 min.) was indicated as 100% activity.
3) The supernatant was diluted in various ratios and the reactivities of the antibody were determined in the concentrations where the reactivities were remarkably decreased by the further dilution.
4) smooth muscle

Table 4

Tissue Specificity of Monoclonal Antibody HBM-1

Determined by Dot Blot Method.

| Tissue from which myosin was obtained | Reacrivity of HBM-1 |
|---|---|
| cerebrum | ++++ |
| cerebellum | +++ |
| liver | ± |
| spleen | — |
| thrombocyte | — |
| aorta[3] | + |
| uterus[3] | + |
| skeletal muscle | — |
| cardiac muscle | — |

1) 5 $\mu$ l of purified myosin solution (0.5 mg/ml) was adsorbed directly to nitrocellulose membrane (equivalent to 2.5 $\mu$ g myosin).

2) 1/50 diluted supernatant was used for the measurement.

3) smooth muscle

(b) Subclass of monoclonal antibody HBM-1 was determined to be IgG1 by using a subclass kit available from Binding-Site, Co., Ltd.

(c) Antigenic binding site of myosin to the monoclonal antibody HBM-1

The heavy chain component of myosin reacted with the monoclonal antibody HBM-1, while a light chain component did not react with the antibody. Further, the monoclonal antibody recognized a specific portion lying on the tail region of a heavy chain of myosin.

(d) Monoclonal antibody HBM-1 could recongize myosins fixed by an acid, formalin, ethanol, or heating, therefore, HBM-1 can recognize myosins in a preparation generally used in immunohistochemistry.

**Claims**

1. A monoclonal antibody specifically reacting with myosin isozymes of human brain tissue.

2. The monoclonal antibody HBM-1 according to claim 1.

3. A monoclonal antibody specifically reacting with myosin isozymes of bovine brain tissue.

4. The monoclonal antibody BM-1 according to claim 3.

5. The monoclonal antibody BM-2 according to claim 3.

6. A monoclonal antibody reacting with several kinds of myosins including myosins present in bovine brain.

7. The monoclonal antibody BM-3 according to claim 6.

8. The monoclonal antibody BM-4 according to claim 6.

9. A method for diagnosing a cerebral nervous disease, brain death, or brain contusion comprising administering to a mammal a monoclonal antibody according to any one of claims 1 to 9.

10. A hybridoma producing a monoclonal antibody specifically reacting with myosins isozymes of human brain tissue.

11. The hybridoma HBM-1 according to claim 10.

12. A method for preparing monoclonal antibody HBM-1 comprising the step of culturing hybridoma HBM-1 in a culture medium.

13. A hybridoma producing a monoclonal antibody specifically reacting with myosins isozymes of bovine brain tissue.

14. The hybridoma BM-1 according to claim 13.

15. The hybridoma BM-2 according to claim 13.

16. A method for preparing monoclonal antibody BM-1 or BM-2 comprising the step of culturing hybridoma BM-1 or BM-2 in a culture medium.

17. A hybridoma producing a monoclonal antibody reacting with several kinds of myosins including myosins present in bovine brain.

18. The hybridoma BM-3 according to claim 17.

19. The hybridoma BM-4 according to claim 17.

20. A method for preparing monoclonal antibody BM-3 or BM-4 comprising the step of culturing hybridoma BM-3 or BM-4 in a culture medium.